Europäisches Patentamt

European Patent Office

Office européen des brevets

(11) Publication number: **0 272 113**
**A2**

# EUROPEAN PATENT APPLICATION

(21) Application number: **87311102.5**

(22) Date of filing: **16.12.87**

(51) Int. Cl.⁴ **C12P 21/00** , C07K 15/00 , G01N 33/574 , G01N 33/577

---

The applicant has filed a statement in accordance with Rule 28 (4) EPC (issue of a sample only to an expert). Accession number(s) of the deposit(s): ECACC 86112101.

(30) Priority: **18.12.86 JP 302410/86**

(43) Date of publication of application:
**22.06.88 Bulletin 88/25**

(84) Designated Contracting States:
**DE FR GB**

(71) Applicant: **KYOWA HAKKO KOGYO CO., LTD.**
**6-1, Ohte-Machi Itchome**
**Chiyoda-ku Tokyo(JP)**

(72) Inventor: **Yoshida, Hajime**
**c/o Patent Department Kyowa Hakko Kogyo**
**Co. Ltd.**
**6-1 Ohtemachi Itchome Chiyoda-ku**
**Tokyo(JP)**
Inventor: **Furuya, Akiko**
**c/o Patent Department Kyowa Hakko Kogyo**
**Co. Ltd.**
**6-1 Ohtemachi Itchome Chiyoda-ku**
**Tokyo(JP)**

(74) Representative: **Lambert, Hugh Richmond et al**
**D. YOUNG & CO. 10 Staple Inn**
**London, WC1V 7RD(GB)**

---

(54) **Anti-human cancer monoclonal antibodies.**

(57) Monoclonal antibodies are disclosed capable of specifically reacting with human colorectal cancer cells and non-reactive with normal colorectal cells, and useful in the diagnosis of human colorectal cancer.

EP 0 272 113 A2

# ANTI-HUMAN CANCER MONOCLONAL ANTIBODIES

The present invention relates to monoclonal antibodies useful in the immunohistological diagnosis of cancer in humans, and specifically colorectal cancer.

Carcinoembryonic antigen (CEA) is a known marker for colorectal cancer, and methods for the detection of colorectal cancer using anti-CEA serum (polyclonal antibody) and anti-CEA monoclonal antibodies have been proposed. However, anti-CEA monoclonal antibodies react with not only colorectal cancer but also many other digestive system cancers and organic cancers, and thus the specificity of anti-CEA monoclonal antibodies for colorectal cancer is not high. NS19-9 established by using a human colorectal cancer cell line as the antigen has recently been used as a monoclonal antibody specific to pancreatic cancer, because it turned out that NS19-9 reacts with pancreatic cancer, bile duct cancer or biliary tract cancer rather than colorectal cancer. [Pro. Natl. Acad. Sci., 76, 1438 (1979)].

In accordance with the present invention a monoclonal antibody has been produced which is capable of reacting with colorectal cancer with a high degree of specificity and at high frequency. The monoclonal antibody is thus potentially very useful in the diagnosis and treatment of colorectal cancer.

More particularly, the monoclonal antibody produced in accordance with this invention is an antibody of the $IgG_1$ class having a high specificity for colorectal cancer, as compared with other human cancer cells, and which is substantially non-reactive with CEA.

Monoclonal antibodies according to this invention may be obtained by immunizing a mammalian animal, preferably mice, with human colorectal cancer tissue membrane preparations, fusing spleen cells from the immunized animal with myeloma cells, preferably from a murine myeloma cell line, to generate hybridomas, selecting from among the hybridomas obtained a hybridoma clone producing a monoclonal antibody having a high specificity to human colorectal cancer, and cultivating the selected hybridoma either in a suitable culture medium or administering the hybridoma to a host animal, e.g. a mouse, to thereby cause hybridoma cell propagation in the ascitic fluid, followed by separation, from the culture or ascitic fluid, of the desired antibody.

A more detailed protocol for the production of the monoclonal antibody according to the present invention is as follows:

## (1) Immunization of animal and preparation of antibody-producing cells

Mice of 3 to 10 weeks old, preferably 8 weeks old, are immunized with human colorectal cancer cells, tissues or membrane preparations derived from such tissues to cause mice to generate antibody-producing cells in the spleen, lymph node and peripheral blood. Mice that have immunological tolerance as a result of pretreatment with normal human colorectal cells should preferably be used. The immunization is generally performed by administering human colorectal cancer cells ($10^6$ to $10^7$ cells per animal), human colorectal cancer tissues, or membrane preparations (membrane fragments) derived from such tissues (10 to 500 μg per animal) together with an appropriate adjuvant (e.g. Freund's complete adjuvant, or aluminum hydroxide gel plus B. pertussis vaccine) to the mice subcutaneously, intravenously or intraperitoneally. Thereafter, the antigen administration is repeated 2 to 5 times at 1 to 2 week intervals. Three to seven days after each immunization, the blood is sampled from the eyeground venous plexus and the serum of each sample is tested as to determine whether it reacts with human colorectal cancer by the enzyme-linked immunosorbent assay (ELISA) given below, as disclosed in Kosomenekisokuteiho (enzyme immunoassay technique) published by Igaku Shoin, Tokyo 1976, for instance.

The normal human colorectal tissues and human colorectal cancer tissues used may be obtained from autopsies or surgical operations. The tissues may immediately frozen and stored at -80°C. For membrane preparation, the tissues are thawed at 4°C in PBS containing 1 mM phenylmethyl sulfonyl fluoride. After mincing they are disrupted with an ultra disperser (LK-21; Yamato, Tokyo, Japan) and homogenized with a Teflon-glass homogenizer (Teflon is a Registered Trade Mark). The homogenate is centrifuged at 100,000 x g, and then the pellet is resuspended at 1 mg protein in 1 mℓ of PBS and stored at -80°C.

Enzyme-linked immunosorbent assay (ELISA)

The membrane preparations of normal or cancer cells or tissues (membrane fragment fraction containing 10 to 1,000 µg of proteins per ml) are distributed into wells of a 96-well plate for EIA (product of Flow Laboratories) (100 to 200 µl per well). After allowing the membrane preparations to stand overnight for two nights at 4°C, the supernatant is removed from the plate and then the plate is washed well with deionized water or phosphate-buffered saline (PBS; 1.83 g of disodium phosphate, 0.21 g of monopotassium phosphate and 7.65 g of sodium chloride in each liter of distilled water, pH 72.). Then, 1% BSA (bovine serum albumin)-PBS is distributed into the wells (100 to 200 µl per well) and protein-binding sites remaining on each well are blocked by allowing the plate to stand overnight for two nights at 4°C. After discarding the BSA-PBS, the wells are washed well with deionized water or PBS. Samples (mouse sera, hybridoma culture supernatants, or roughly purified monoclonal antibodies; each as the first antibody) are diluted with BSA-PBS and the dilutions are distributed into the wells (100 µl per well), followed by overnight standing at 4°C. After washing the wells once with deionized water and then 6 times with 2 M NaCl solution, a 100-fold dilution of the rabbit anti-mouse immunoglobulin IgG-peroxidase conjugate (product of DAKO and distributed by Kyowa Medex; used as the second antibody) is distributed into the wells (100 µl per well). The plate is then allowed to stand at room temperature for 2 hours.

After washing well with PBs, an ABTS substrate solution [prepared by dissolving 550 mg of 2,2'-azinobis (3-ethylbenzothiazoline-6-sulfonic acid) diammonium salt in 1 liter of 0.1 M citrate buffer (pH 4.2) and adding, just prior to use, hydrogen peroxide to a concentration of 1 µl/ml] is applied and the color developed is measured in terms of the absorbance $OD_{415nm}$. Those mice that strongly react with the colorectal cancer cells, tissues or membrane preparations thereof are used as human colorectal cancer-immunized mice, namely as the source of antibody-producing cells for the hybridoma production.

When cells as such are used as the antigen in performing ELISA, the target cells are cultivated on a Falcon 3072 plate, 0.25% glutaraldehyde-PBS is added, and after allowing it to stand at room temperature for 1-2 hours, the plate is washed well with PBS. Then, 100-200 µl of 1% BSA-PBS is added, and after allowing the plate to standing for 2 hours, the plate is washed well with deionized water or PBS and submitted to antibody titer determination, which is conducted in the same manner as the case where an ordinary antigen-coated plate is used.

For submitting to cell fusion, human colorectal cancer cells, tissues or membrane preparations are intraperitoneally administered to the immunized mice in a dose of 2 to 5 x $10^6$ cells per animals or 20 to 400 µg per animal 3-4 days prior to the fusion treatment. The spleen is extirpated, cut into fragments in MEM (product of Nissui Pharmaceutical), loosened up with a pair of forceps, and centrifuged at 1,200 rpm for 5 minutes. The supernatant is discarded, and the sediment is deprived of erythrocytes by treatment with Tris-ammonium chloride buffer (pH 7.65) for 1-2 minutes, washed three times with MEM, and used as the spleen cells for fusion.

(2) Preparation of myeloma cells

A mouse-derived established myeloma cell line is preferably used. Usable examples of such cell line include the 8-azaguanine resistant murine (BALB/c-derived) myeloma cell lines P3-X63Ag8-U1 (P3-U1) [Current Topics in Microbiology and Immunology-1] P3-NSI/1-Ag4.1 (NS-1) [European J. Immunology, 6, 511-519 (1976)], SP2/0-Ag14 (SP-2) [Nature, 276, 269-270 (1978)], P3-X63-Ag8 653 (653) [J. Immunology, 123, 1548-1550 (1979)] and P3-X63-Ag8 (X63) [Nature, 256, 495-8-azaguanine medium [normal medium prepared by adding, to RPMI-1640 medium, glutamine (1.5 mM), 2-mercaptoethanol (5 x $10^5$ M), gentamycin (10 µg/ml) and fetal calf serum (FCS; product of CSL) (10%), with further supplementation with 8-azaguanine (15 µg/ml)]. The cell line selected for cell fusion is transferred to normal medium 3-4 days before fusion to ensure the cell count of not less than 2 x $10^7$ on the day of fusion.

(3) Cell fusion

Spleen cells prepared as in (1) and the myeloma cells obtained as in (2) are washed well with MEM or PBS, mixed in a cell number ratio of spleen cells: myeloma cells = 5 to 10:1 and then subjected to centrifugation (1,200 rpm, 5 minutes). The supernatant is discarded and the cell sediment is loosened up. With stirring at 37°C, a mixture of 2 g of polyethylene glycol 1000 (PEG-1000), 2 ml of MEM and 0.7 ml of dimethyl sulfoxide is added in an amount of 0.2-1 ml per $10^3$ spleen cells, and MEM is added until the whole volume is made up to be 50 mℓ after several additions of 1-2 mℓ of MEM at 1-to 2-minute intervals. After centrifugation (900 rpm, 5 minutes), the supernatant is discarded and the cell sediment is loosened gently. To the cells is added 100 mℓ of normal medium (RPMI-1640 with 10% FCS). The cells are gently suspended in the medium with a measuring pipette.

The suspension obtained is distributed, in 1 mℓ-portions, into the wells of a 24-well incubation plate. Incubation is carried out in a 5% $CO_2$ incubator at 37°C for 24 hours. HAT medium [normal medium supplemented with hypoxanthine ($10^4$ M), thymidine ($1.5 \times 10^5$ M) and aminopterine ($4 \times 10^7$ M)] is added to the incubation plate (1 mℓ per well) and incubation is conducted for a further 24 hours. Thereafter, 1 mℓ of the culture supernatant is discarded and the same volume of fresh HAT medium is added at 24-hour intervals for 2 days. The incubation in the $CO_2$ incubator at 37°C is continued for 10-14 days.

From those wells in which fused, colony-forming grown cells are found, 1 mℓ of the supernatant is discarded and the same volume of HT medium (HAT medium minus aminopterine) is added, followed by medium replacement with fresh portions of HT medium at 24-hours intervals for 2 days.

After 3-4 days of cultivation in HT medium, a portion of the culture supernatant is collected and assayed for antibody titer relative to human colorectal cancer by the above-mentioned ELISA. Simultaneously, the reactivities with normal human cells or tissues and membrane preparations thereof, among others, are also determined by a similar method, and those wells for which selective reactivity with human colorectal cancer cells or tissues or membrane preparations thereof is shown are selected. For the wells showing strong reactivity with human colorectal cancer cells or tissues or membrane preparations thereof but no reactivity with normal human cells or tissues or membrane preparations thereof, among others, cloning is repeated twice by the limiting dilution techniques. In this way, those clones for which high antibody titer values are stably obtainable relative human colorectal cancer cells or tissues or membrane preparations thereof are selected as anti-human colorectal cancer monoclonal antibody-producing hybridoma cell lines.


(4) Preparation of monoclonal antibodies

Eight-to ten-week-old female nude mice treated with pristane [intraperitoneally administered with 0.5 mℓ of 2,6,10,14-tetramethylpentadecane (pristane) and fed for 2 weeks] are intraperitoneally injected with the anti-human colorectal cancer monoclonal antibody-producing hybridoma cells obtained in procedure (3) above at a dose of $2\text{-}4 \times 10^6$ cells per animal. In 10-21 days, the hybridoma cells produce ascites carcinoma in the mice. The ascitic fluid is collected from such mice, centrifuged (3,00 rpm, 5 minutes) to remove solids, subjected to salting out with 50% ammonium sulfate, dialyzed against 0.04 M phosphate buffer (pH 8.0) supplemented with 0.03 M NaCl, and passed through DE52 ® (product of Whatman) column. An IgG fraction is collected and used as a purified monoclonal antibody.

The isotype of the antibody is determined by Ouchterlony's method (double immunodiffusion) [Seibutsukagaku Jikkenho (Methods in Experimental Biochemistry), vol. 15, Introduction to Experimental Immunology, p. 74, Gakkai Shuppan Center, 1981].

The quantity of protein is estimated by the Folin's method, followed by calculation based on the absorbance at 280 nm [1.4 ($OD_{280}$) approximately corresponds to 1 mg of immunoglobulin per mℓ].

The monoclonal antibodies thus obtained are evaluated for specificity characteristics based on (1) the reactivities with normal and cancer tissues and membrane preparations thereof derived from a variety of human organs obtained from a plurality of subjects, 92) the reactivities with a variety of normal human or cancel cell lines or human fetal cell line, or membrane preparations derived therefrom, (3) the reactivity with the hitherto known cardioembryonic antigen (CEA), and the like as determined by an appropriate assay technique, such as ELISA, immunohistological staining method (ABC method), etc. Those monoclonal antibodies that react with human colorectal cancer and do not exhibit reactivity with the other antigens in any evaluation test are selected.

The thus-obtained monoclonal antibodies which react specifically with human colorectal cancer cells, are expected to be useful in the treatment of colorectal cancer which comprises administering the antibodies as they are in the form of the so-called immunotoxins, namely conjugates with anticancer agents or toxins, to cancer patients.

## (5) Antigen analysis

In performing the above-mentioned ELISA, and immunohistological staining method, the antigens (colorectal cancer membrane preparations, cultured colorectal cancer cell lines, colorectal cancer tissues) are pretreated with reagents such as enzymes (e.g. neuraminidase, protease) or periodic acid and then reacted with the monoclonal antibodies. The subsequent comparison for differences in reactivity with the monoclonal antibodies between the original antigens without such pretreatment and the antigens pretreated in the above manner can elucidate the chemical characteristics of the antigenic sites which the monoclonal antibodies recognise. That is, if the antigenicity disappears upon treatment with neuraminidase, it is assumed that sialic acids are associated with the antigenic determinants. If the antigenicity disappears upon treatment with protease and trypsin, it is assumed that proteins are associated with the antigenic determinants. If the antigenicity disappears upon periodic acid treatment, sugar chains are presumably associated with the antigenic determinants. If the antigenicity disappears upon $\alpha$-L-fucosidase treatment, fucose at the terminal of sugar chains is presumably associated with the antigenic determinants.

The production of monoclonal antibodies according to the present invention is described in further detail in the following Examples.

## Example 1

### (1) Preparation of antibody-producing cells

Normal human colorectal tissue membrane preparations were administered intravenously to new-born BALB/c mice (purchased from Shizouka Agricultural Cooperative Association for Laboratory Animals) within 24 postnatal hours at a dose of 100 $\mu$g of proteins per animal. After the lapse of 8 weeks, the mice were intraperitoneally administered with human colorectal cancer membrane preparations (100 $\mu$g of proteins per animal) together with aluminum hydroxide gel (2 mg per animal) and killed B. pertussis vaccine (1 x 10$^9$ per animal), followed by 3 to 5 immunizations with the same antigen without adjuvant at a dose of 100 $\mu$g per animal on the protein basis at 1 to 2 week intervals. From among these immunized mice, those mice whose antisera intensely reacted with human colorectal cancer cells or tissues or membrane preparations derived therefrom were selected, and spleen cells were prepared from such mice and submitted to cell fusion.

### (2) Preparation of myeloma cells

The 9-azaguanine-resistant murine myeloma cell line P3-U1 was cultivated in normal medium to thereby secure not less than 2 x 10$^7$ cells at the time of cell fusion, and submitted to cell fusion as a parent strain.

### (3) Hybridoma production

The spleen cells and myeloma cells obtained in (1) and (2) respectively were used in a ratio of 5:1 and subjected to fusion according to the procedure mentioned hereinabove. After cultivation in HAT medium at 37°C under 5% CO$_2$ for 14 days, fused cells were selected, and after change of the medium to HT medium, cultivation was continued. Based on the results of anti-human colorectal cancer antibody titer determination, active wells were selected, and after change of the medium to normal medium, cloning was repeated twice. The hybridoma cell line ACC-574 having no reactivity with normal human cells or tissues, or other cancers and having specific reactivity with human colorectal cancer cells, as determined by various assay methods, was thus selected.

The hybridoma cell line ACC-574 has bee deposited under the terms of the Budapest Treaty with the European Collection of Animal Cell Cultures, Great Britain, on 21th November, 1986 as ECACC Deposit No. 86112101.

(4) Monoclonal antibody purification

Pristane-treated 8-week-old female nude mice were intraperitoneally injected with the hybridome cell line ACC-574 obtained in (3) at a dose of 4 x 10⁶ cells per animal. In 10-21 days, the hybridoma produced ascites carcinoma. The ascitic fluid was collected from ascitic fluid-bearing mice (5-10 ml per animal), deprived of solids by centrifugation (3,00 rpm, 5 minutes), subjected to salting out with 40% ammonium sulfate, dialyzed against 0.04 M phosphate buffer (pH 8.0) supplemented with NaCl (0.03 M), and passed through a DE52® (product of Whatman) column (bed volume 50 ml) at a flow rate of 20-30 ml/hr. An IgG fraction was collected and used as purified antibody.

(5) Specificity of ACC-574

The specificity of the thus-obtained anti-human colorectal cancer monoclonal antibody ACC-574 was investigated using enzyme-linked immunosorbent assay.

The enzyme-linked immunosorbent assay (ELISA) was carried out as follows.

In the case of membrane preparations from tissues as a target, a solution of 0.1 mg/ml membrane preparations from tissues was distributed in 50 μl portions into the wells of a 96-well plate for EIA (purchased from Linbro). After allowing it to stand at 37°C for 2 hours or at 4°C overnight, the plate in which the membrane preparations from tissues had been fixed was washed with PBS. Then PBS supplemented with 10% fetal calf serum was distributed into the wells (100 μl per well). The plate in which the active residues of the fixed membrane preparations from tissues had been protected was washed with PBS. The first antibody (ACC-574) was distributed into the wells (50 μl per well), followed by allowing the plate to stand at 37°C for 1-2 hours or at 4°C overnight to carry out the reaction between the target and the antibody.

After washing five times with PBS supplemented with 0.05% Tween-20® (purchased from Wako Pure Chemicals) to remove the unreactive antibodies, peroxidase-labeled rabbit anti-mouse immunoglobulin (purchased from Miles-Yeda; 200-fold dilution) as the second antibody was distributed into the wells (50 μl per well), and the reaction was carried out at 37°C for 1 hour. After washing five times with PBS supplemented with 0.05% Tween-20® and three times with deionized water, the ABTS substrate solution was added (50 μl per well) and the reaction was allowed to proceed and then terminated by adding 5% sodium lauryl sulfate solution (50 μl per well).

In the case of cultivated cell lines as a target, the cell lines were cultivated in the wells of a 96-well plate for cultivation (purchased from Linbro). After the cells were confluent in the plate, the immune reaction was allowed to proceed in a similar manner to the case of the membrane preparations from tissues described above except that the reactions of the first antibody and of second antibody concerned were carried out at room temperature for 30 minutes. After the color development, the reaction was terminated by transferring the reaction mixture into a 96-well plate for analysis.

In the case of CEA, the immune reaction was carried out in a similar manner to the case of a membrane preparations from tissues except that CEA instead of the membrane preparations from tissues was used.

For each case, the absorbance at 415 nm was measured, putting the absorbance at 490 nm as the control.

## Table 1
### Binding activity (ELISA)

| | Target | Antibody ACC-574 positive result/ test samples |
|---|---|---|
| Membrane preparations from tissues | Gastric cancer | 2/30 |
| | Pancreatic cancer | 1/4 |
| | Colorectal cancer | 4/4 |
| | Tissues derived from normal stomach | 0/6 |
| | Tissues derived from normal colorectum | 0/4 |
| Cultured cell lines | Gastric cancer | 2/5 |
| | Pancreatic cancer | 1/2 |
| | Colorectal cancer | 2/2 |
| | Lung cancer | 1/5 |
| | Fetal skin | 0/1 |
| Antigen | CEA | 0/1 |

As shown in Table 1, ACC-574 has high specificity for colorectal cancer. But since ACC-574 is non-reactive with CEA, ACC-574 is different from anti-CEA antibody.

Example 2

Formalin-fixed and paraffin-embedded tissue sections of various normal adult or fetal organs, or cancer tissues sliced to a thickness of 5 μm with microtome were mounted on glass slides coated with egg white albumin, dewaxed in xylene, and gradually hydrated with aqueous alcohol.

After washing with deionized water for 5 minutes, endogenous peroxidase was blocked by immersion in 0.3% (wt/vol) hydrogen peroxide in an absolute methanol at room temperature for 30 minutes. The sections were washed with PBS for 20 minutes and incubated with diluted normal horse serum at room temperature for 20 minutes.

Excess serum was soaked up and the anti-human colorectal cancer monoclonal antibody ACC-574 (10 μg/mℓ) was added as the first antibody. The glass was allowed to stand at room temperature for 30 minutes and then washed with PBS. Then a diluted biotin-labeled antibody (Biotin-labeled rabbit anti-IgG antibody) was added to the sections and the glass was allowed to stand for 30 minutes. After washing, an avidin-biotin-peroxidase (product of Vector) was added to the sections and the glass was allowed to stand for 30 minutes. After washing well, the sections were incubated for 2 minutes in a peroxidase substrate solution (mixture of 0.02% hydrogen peroxidase and 0.1% diaminobenzidine tetrahydrochloride in 0.1 M Tris-hydrochloride buffer, pH 7.2) and the reaction was stopped by putting the glass into ice-cold water. The sections were counterstained with hematoxylin, dehydrated in alcohol/water and xylene, settled with Canada balsam and submitted to microscopic examination.

The results are shown in Table 2.

## Table 2

| Tissue | Positive result/<br>Test samples | Positive rate<br>(%) |
|---|---|---|
| Gastric cancer | 5/11* | 45.5 |
| Colorectal cancer | 5/8 | 62.5 |
| Pancreatic cancer | 1/1* | 100.0 |
| Uterine cancer | 3/5* | 60.0 |
| Bladder cancer | 0/1 | 0 |
| Neuroblastoma | 0/1 | 0 |
| Palatine cancer | 0/1 | 0 |
| Hepatoma | 0/1 | 0 |
| Bile duct cancer | 0/1 | 0 |
| Laryngeal cancer | 0/1 | 0 |

### * ACC-574 weakly reacted with a part of the cancer cells.

As shown in Table 2, while ACC-574 weakly reacted with a part of gastric cancer, pancreatic cancer and uterine cancer tissues, ACC-574 strongly reacted with colorectal cancer tissue with high frequency. With respect to normal adult and fetal tissues such as heart, pancreas, stomach, large intestine, kidney, thyroid gland, brain, spleen, liver, small intestine, ACC-574 reacted with a part of macrophage and polymorphonuclear leucocyte in the normal adult and fetal tissues, and did not react with the remainder of the tissues.

Example 3

For analysing the antigens which the monoclonal antibody ACC-574 recognizes, colorectal cancer tissue membrane preparations were treated with the enzymes and reagent described below and then examined for the reactivity with ACC-574.

Enzymes and reagent

Trypsin (2.5% solution; Gibco)

0.25% in PBS

Neuraminidase (Boehringer Mannheim)

0.1 U/ml in 0.1 M acetate buffer (pH 4.5)-3 mM $CaCl_2$

α-L-Fucosidase (Boehringer Mannheim)

0.1 U/ml in 0.1 M phosphate buffer (pH 6.3)

Protease (Sigma)

10 U/mℓ in 0.1 M phosphate buffer (pH 7.2)
NaIO₄ (Wako Pure Chemical Industries)
50 mM in PBS

The colorectal cancer tissue membrane preparations (100 μg proteins per mℓ) were distributed, in 50 μℓ portions, into the wells of a plate of EIA (purchased from Linbro). After overnight standing at 4°C, the plate was washed three times with PBS. Then, 1% BSA-PBS was distributed into the wells (200 μℓ per well). The plate was allowed to stand at room temperature for 30 minutes to 2 hours and then washed three times with PBS. One of the above enzymes or reagent was distributed into the wells (50 μℓ per well). After 1 hour of reaction at 37°C, the plate was washed five times with PBS. Then, the monoclonal antibody ACC-574 (10 μg/mℓ) was distributed into the wells (50 μℓ per well), followed by allowing the wells to stand at 4°C for overnight.

After washing five times with Tween 20 (Wako Pure Chemical Industries)-PBS, peroxidase-labeled antimouse IgG (400-fold dilution) was added in an amount of 50 μℓ per well, and the reaction was allowed to proceed at room temperature for 2 hours. The plate was washed five times with Tween 20-PBS, an ABTS substrate solution was added in an amount of 100 μℓ and, after 30 minutes of reaction, the absorbance was measured at 415 nm.

Inhibition ratio is calculated by the following equation.

$$\text{Inhibition ratio} = \frac{A - B}{A} \times 100\%$$

wherein A: no treatment
B: treatment with enzymes or a reagent.

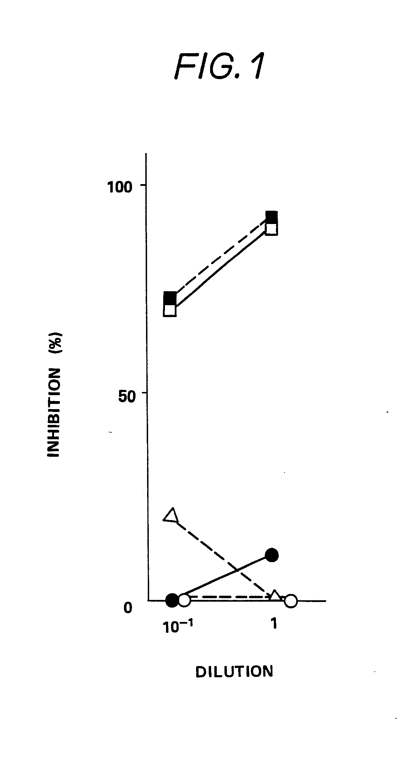

The results are presented graphically in Figure 1 of the accompanying drawings. In the drawing the symbols are used as follows: ● for treatment with 0.1 U/ml of neuraminidase, Δ for treatment with 0.1 U/ml for alpha-L-fucosidase, □ for treatment with 0.25% trypsin, ■ for treatment with 10 U/ml of protease, and ○ for treatment with 50 mM NaIO₄.

As shown in Figure 1, the antigenicity disappeared completely upon treatment with protease or trypsin. On the basis of these findings, it was estimated that the monoclonal antibody ACC-574 might recognize proteins.

Whilst the invention has been described in terms of a particular monoclonal antibody, and a particular cell line capable of expressing that antibody, present day technology is such that it is now feasible to recover cDNA or cDNA sequences encoding for a particular protein and to insert such cDNA or cDNA sequences by recombinant DNA technology into a host thereby transforming that host into a transformant capable of expressing that protein. Thus the present invention extends to cDNA and cDNA sequences encoding for monoclonal antibodies of the type described, and especially to such cDNA or cDNA sequences when obtained from the genome of the hybridome cell line ACC-574, ECACC No. 86112101, and to transformants transformed with such cDNA and which are capable of expressing the corresponding monoclonal antibody.

## Claims

1. An anti-human cancer monoclonal antibody, characterised in that the antibody is of the IgG₁ class, has a high specificity towards human colorectal cancer cells, as compared with that towards other human cancer cells, and is substantially non-reactive with human carcinoembryonic antigen (CEA).

2. A monoclonal antibody according to claim 1, which is obtained from the hybridoma cell line ACC-574 (ECACC No. 86112101) whether directly, or indirectly by transformation of a host using a genetic sequence derived from ACC-574 and encoding for that antibody, and using that host to express the antibody.

3. A method of producing an anti-human cancer monoclonal antibody which comprises immunizing a mammalian animal with human colorectal cancer cells, human colorectal cancer tissue or membrane preparations thereof, fusing spleen cells from the immunized mammal with mammalian myeloma cells, selecting from the hybridoma cell lines thus obtained a hybridoma cell line capable of producing the required antibody, cultivating the selected hybridoma cell line in a culture medium, or intraperitoneally administering the selected cell line to a mammalian animal, thereby to cause hybridoma cell line propagation in said culture medium or in the ascitic fluid of said mammal, and recovering product monoclonal antibody from the culture medium or from the ascitic fluids of said mammal and containing said propagated cells, characterised in that there is selected as said hybridoma cell line, a hybridoma cell line which produces a monoclonal antibody having a high specificity towards human colorectal cancer cells, as compared with other human cancer cells, thereby to produce an anti-human cancer monoclonal antibody having a high specificity towards colorectal cancer.

4. A method according to claim 3, wherein the mammalian animals used are mice.

5. A method according to claim 3 or 4, wherein the mammalian animals used to produce the initial antibody producing cells are rendered immunologically tolerant by pretreatment with normal human colorectal cells.

6. A hybridoma cell line having the ability to produce a monoclonal antibody according to claim 1.

7. The hybridoma cell line ACC-574, ECACC No. 86112101.

8. An immunohistological method for detecting the presence of human colorectal cancer in a patient, comprising (a) subjecting a tissue sample from the patient to immunoassay with a monoclonal antibody that (1) is specific to and reacts with human colorectal cancer, (2) recognises proteins as antigens, and (3) is non-reactive with normal human colorectal cell; and (b) examining the results for the presence of a positive reaction.

9. An immunohistochemical staining method for the detection of human colorectal cancer in a patient, which comprises applying to a tissue sample from the colorectum of the patient a monoclonal antibody according to claim 1 and detecting the selective reaction of the antibody with any human colorectal cancer cells present in the sample by differential staining as between the cells comprising the reacted antibody and normal human colorectal tissue or cells which are non-reactive with and therefore do not contain the antibody.

10. A method according to claim 8 or 9, wherein the monoclonal antibody used is as defined in claim 2.

11. A cDNA or cDNA sequence encoding for a monoclonal antibody according to claim 1 or 2.

12. A cDNA or cDNA sequence according to claim 11, when derived from the genome of hybridoma cell line ACC-574, ECACC No. 86112101.

13. A transformant transformed with a cDNA or cDNA sequence according to claim 11 or 12, and capable of expressing monoclonal antibody according to claim 1.

14. A method of producing a monoclonal antibody according to claim 1, which comprises culturing a hybridoma cell line according to claim 6, or a transformant according to claim 13, in a culture medium, including the ascitic fluid of a host animal, thereby to accumulate the antibody in the culture medium and recovering the accumulated antibody.

15. A method according to claim 14, wherein there is used hybridoma cell line ACC-574, ECACC No. 86112101, or a transformant obtained using a genetic sequence coding from the antibody and derived from ACC-574, ECACC No. 8612101.

# FIG. 1